(19) Europäisches Patentamt
European Patent Office
Office européen des brevets

(11) Veröffentlichungsnummer: **0 227 588 B1**

(12) **EUROPÄISCHE PATENTSCHRIFT**

(45) Veröffentlichungstag der Patentschrift: **06.11.91**

(51) Int. Cl.$^5$: **C12P 33/00**, C07J 1/00

(21) Anmeldenummer: **86730207.7**

(22) Anmeldetag: **09.12.86**

# (54) Verfahren zur Herstellung von 4-Androsten-3, 17-dion und 1,4-Androstadien-3,17-dion.

(30) Priorität: **13.12.85 DE 3544662**

(43) Veröffentlichungstag der Anmeldung:
**01.07.87 Patentblatt 87/27**

(45) Bekanntmachung des Hinweises auf die Patenterteilung:
**06.11.91 Patentblatt 91/45**

(84) Benannte Vertragsstaaten:
**AT BE CH DE ES FR GB GR IT LI LU NL SE**

(56) Entgegenhaltungen:
**DE-A- 2 703 645**
**FR-A- 2 408 621**
**GB-A- 1 329 387**
**US-A- 3 684 657**

**EXPERIENTIA, Band 24, Nr. 5, Mai 1968, Seite 432, Berkhäuser Verlag, Basel, CH; G. AMBRUS et al.: "Über die Hydrierung im Ringsystem ungesättigter Steroide mit Mycobacterium phlei"**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **Weber, Alfred, Dr.**
**Schützallee 56**
**W-1000 Berlin 37(DE)**
Erfinder: **Kennecke, Mario, Dr.**
**Taubertstrasse 31 f**
**W-1000 Berlin 33(DE)**
Erfinder: **Kurzidim, Johannes, Dr.**
**Brahmsstrasse 11 d**
**W-1000 Berlin 45(DE)**

EP 0 227 588 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft das in den Patentansprüchen gekennzeichnete Verfahren.

Es ist bekannt, daß zahlreiche Mikrooganismen (so zum Beispiel solche der Gattungen Arthrobacter, Brevibacterium, Microbacterium, Protaminobacter, Bacillus, Norcardia, Streptomyces und insbesondere Mycobacterium) die natürliche Fähigkeit besitzen, natürlich vorkommende 3$\beta$-Hydroxy-$\Delta^5$-sterine (wie Cholesterin oder Sitosterin) zu Kohlendioxyd und Wasser abzubauen und daß bei diesem Abbau intermediär 4-Androsten-3,17-dion und 1,4-Androstadien-3,17-dion gebildet werden.

Ferner ist bekannt, daß es mithilfe von Inhibitorzusätzen oder mutierten Mikroorganismen möglich ist, den Abbau der Sterine so zu lenken, daß ein Abbau des gebildeten 4-Androsten-3,17-dions oder 1,4-Androstadien-3,17-dions vermieden wird (siehe deutsche Offenlegungsschriften 15 43 269 und 15 93 327 sowie US-Patentschrift 3,684,657).

Für den Fachmann ist es überraschend, daß unter den bekannten Bedingungen auch die Seitenkette von $\alpha$-Sitosterin abgebaut wird, weil bekannt ist, daß der Seitenkettenabbau von Sterinen durch ein sehr komplexes Fermentsystem bewirkt wird und man nicht erwarten konnte, daß alle am Seitenkettenabbau natürlicher Steroide mitwirkenden Enzyme die Fähigkeit besitzen, auch den Seitenkettenabbau dieser Verbindung zu bewirken. Darüberhinaus konnte man nicht vorhersehen, daß bei diesem Abbau die $\Delta^7$-Doppelbindung des $\alpha$-Sitosterins hydriert und die in 4$\alpha$-Position ständige Methylgruppe abgespalten wird.

Abgesehen von der Verwendung anderer Ausgangsverbindungen, wird das erfindungsgemäße Verfahren unter den gleichen Fermentationsbedingungen durchgeführt, welche man auch bei den bekannten mikrobiologischen Seitenkettenabbau-Reaktionen von Sterinen anwendet.

Erfindungsgemäß wird die Fermentation unter Verwendung der Mikroorganismenkultur durchgeführt, welche man üblicherweise zum Seitenkettenabbau von Sterinen verwendet. Geeigente Kulturen sind beispielsweise zum Seitenkettenabbau von Sterinen befähigte Bakterienkulturen der Gattungen Arthrobacter, Brevibacterium, Microbacterium, Protaminobacter, Streptomyces oder insbesondere der Gattung Mycobacterium. Als geeignete Mikroorganismen seien beispielsweise genannt: Microbacterium lactum IAM-1640, Protaminobacter alboflavus IAM-1040, Bacillus roseus IAM-1257, Bacillus sphäricus ATTC-7055, Norcardia gardneri IAM-105, Norcardia minima IAM-374, Norcardia corallina IFO-3338, Streptomyces rubescens IAM-74 oder insbesondere die Mikroorganismen Mycobacterium avium IFO-3082, Mycobacterium phlei IFO-3158, Mycobacterium phlei (Institut für Gesundheitswesen, Budapest Nr. 29), Mycobacterium phlei ATCC-354, Mycobacterium smegmatis IFO-3084, Mycobacterium smegmatis ATCC-20, Mycobacterium smegmatis (Institut für Gesundheitswesen, Budapest Nr. 27), Mycobacterium smegmatis ATCC-19979 und Mycobacterium fortuitum CBS-49566.

Besonders bevorzugt sind als Mikroorganismen Mycobacterium spec. NRRL B-3805, Mycobacterium spec. NRRL B-3683, Mycobacterium phlei NRRL B-8154 und Mycobacterium fortuitum NRRL B-8153 mit deren Hilfe $\alpha$-Sitosterin ohne Verwendung zusätzlicher die 9$\alpha$-Hydroxylierung hemmender Inhibitoren durchgeführt werden kann.

Unter den für diese Mikroorganismen üblicherweise verwendeten Kulturbedingungen werden in einem geeigneten Nährmedium unter Belüften, Submerskulturen angezüchtet. Dann setzt man den Kulturen das Substrat (in einem geeigneten Lösungsmittel gelöst oder vorzugsweise in emulgierter Form) zu und fermentiert, bis eine maximale Substratumwandlung erreicht ist.

Geeignete Substratlösungsmittel sind beispielsweise Methanol, Äthanol, Glykolmonomethyläther, Dimethylformamid oder Dimethylsulfoxyd). Die Emulgierung des Substrats kann beispielsweise bewirkt werden, indem man dieses in mikronisierter Form oder in einem mit Wasser mischbaren Lösungsmittel (wie Methanol, Äthanol, Aceton, Glykolmonomethyläther, Dimethylformamid oder Dimethylsulfoxyd) gelöst unter starker Turbulenz in (vorzugsweise entkalktem) Wasser, welches die üblichen Emulgationshilfen enthält eindüst. Geeignete Emulgationshilfen sind nichtionogene Emulgatoren, wie zum Beispiel Äthylenoxyaddukte oder Fettsäureester von Polyglykolen. Als geeignete Emulgatoren seien die handelsüblichen Netzmittel Tegin$^{(R)}$, Tween$^{(R)}$ und Span$^{(R)}$ beispielsmäßig genannt.

Die optimale Substratkonzentration, Substratzugabezeit und Fermentationsdauer ist von der Struktur des verwendeten Substrates und der Art des verwendeten Mikroorganismus abhängig. Diese Größen müssen, wie dies bei mikrobiologischen Steroidumwandlungen allgemein erforderlich ist, im Einzelfall durch Vorversuche, wie sie dem Fachmann geläufig sind, ermittelt werden.

Die nach dem erfindungsgemäßen Verfahren herstellbaren 4-Androsten-3,17-dion-Derivate der allgemeinen Formel I sind bekanntlich wertvolle Zwischenprodukte die heute gewerbsmäßig zur Synthese phamakologisch wirksamer Steroide verwendet werden.

Die nachfolgenden Beispiele dienen zur Erläuterung des erfindungsgemäßen Verfahrens.

Beispiel 1

Ein 2 l-Erlenmeyerkolben mit 500 ml sterilem Nährmedium, enthaltend
1 % Hefeextrakt
0,45 % Dinatriumhydrogenphosphat
0,34 % Kaliumdihyrogenphosphat und
0,2 % Tween (R)80
-eingestellt auf pH 6,7 -
wird mit einer Suspension einer Mycobacterium spec. NRRL B-3805-Trockenkultur beimpft und 3 Tage lang bei 30° C mit 190 Umdrehungen pro Minute geschüttelt.

Erlenmeyerkolben (500 ml) mit jeweils 100 ml sterilem Nährmedium, enthaltend
2,5 % Cornsteep liquor
0,3 % Diammoniumhydrogenphosphat
0,25 % Sojamehl und
0,25 % Tween (R)80
eingestellt auf pH 7,0 -
werden mit jeweils 5 ml der Mycobacterium spec.-Anzuchtskultur beimpft und 24 Stunden lang bei 30° C mit 220 Umdrehungen pro Minute geschüttelt. Dann setzt man jeder Kultur 100 mg α-Sitosterin in 3,0 ml Dimethylformamid gelöst zu und fermentiert weitere 96 Stunden lang bei 30° C.

Die vereinigten Kulturen werden mit Äthylenchlorid extrahiert, der Extrakt im Vakuum eingeengt, der Rückstand durch Chromatographie über eine Kieselgelsäule gereinigt und man erhält nach Umkristallisation aus Dissopropyläther 160 mg 4-Androsten-3,17-dion.

Daneben werden 385 mg nicht umgesetztes α-Sitosterin zurückgewonnen.

Beispiel 2

Unter den Bedingungen des Beispiels 1, jedoch unter Verwendung von Mycobacterium spec. NRRL B-3683 werden insgesamt 1000 mg α-Sitosterin umgesetzt, aufbereitet und man erhält neben 630 mg unumgesetzten α-Sitosterin 140 mg 1,4-Androstadien-3,17-dion.

**Patentansprüche**

1. Verfahren zur Herstellung von 4-Androsten-3,17-dion und 1,4-Androstadien-3,17-dion der allgemeinen Formel

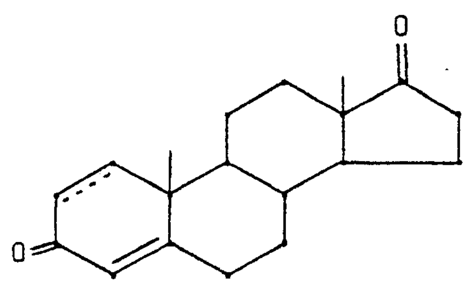

worin
..... eine Einfachbindung oder eine Doppelbindung symbolisiert,
dadurch gekennzeichnet, daß man α-Sitosterin

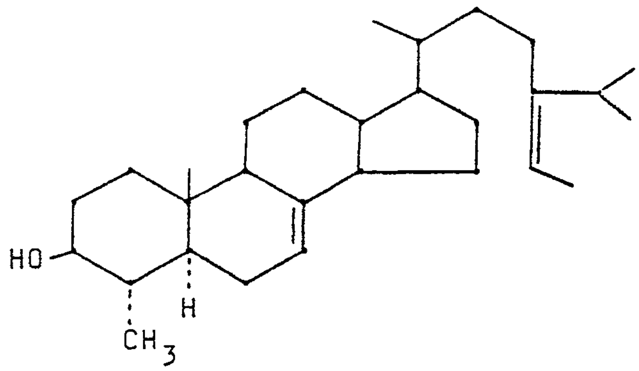

mit einer Kultur eines zum Seitenkettenabbau von Sterinen befähigten Mikroorganismen-Stammes fermentiert.

2. Verfahren zur Herstellung von 4-Androsten-3,17-dion und 1,4-Androstadien-3,17-dion gemäß Patentanspruch 1, dadurch gekennzeichnet, daß man α-Sitosterin mit einem zum Seitenkettenabbau von Sterinen befähigten Mikroorganismen-Stamm der Gattung Mycobacterium oder Norcardia fermentiert.

3. Verfahren zur Herstellung von 4-Androsten-3,17-dion und 1,4-Androstadien-3,17-dion gemäß Patentanspruch 1 und 2, dadurch gekennzeichnet, daß man α-Sitosterin mit Mycobacterium spec. NRRL B-3805, Mycobacterium spec. NRRL B-3683, Mycobacterium phlei NRRL B-8154 oder Mycobacterium fortuitum NRRL B-8153 fermentiert.

**Claims**

1. Process for the preparation of 4-androstene-3,17-dione and 1,4-androstadiene-3,17-dione of the general formula

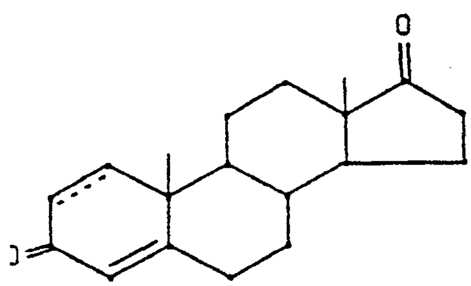

wherein
..... represents a single bond or a double bond, characterised in that α-sitosterol

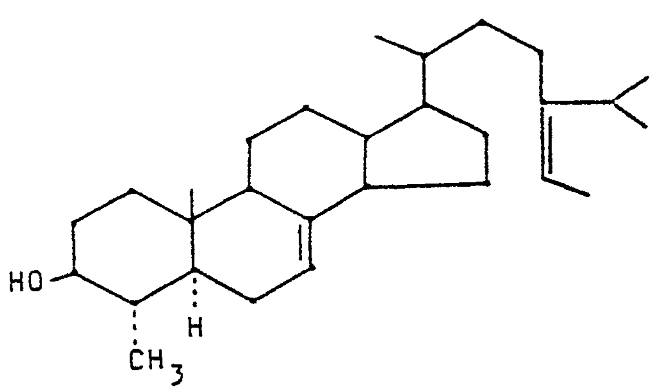

is fermented with a culture of a microorganism strain that is capable of breaking down the side-chains of sterols.

2. Process for the preparation of 4-androstene-3,17-dione and 1,4-androstadiene-3,17-dione according to patent claim 1, characterised in that α-sitosterol is fermented with a microorganism strain of the genus Mycobacterium or Norcardia that is capable of breaking down the side-chains of sterols.

3. Process for the preparation of 4-androstene-3,17-dione and 1,4-androstadiene-3,17-dione according to patent claims 1 and 2, characterised in that α-sitosterol is fermented with Mycobacterium spec. NRRL B-3805, Mycobacterium spec. NRRL B-3683, Mycobacterium phlei NRRL B-8154 or Mycobacterium fortuitum NRRL B-8153.

## Revendications

1. Procédé pour préparer l'androstène-4 dione-3,17 et l'androstadiène-1,4 dione 3,17, qui répondent à la formule générale :

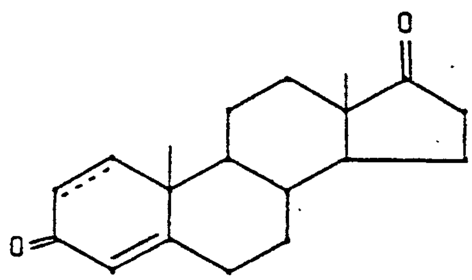

dans laquelle ...... représente symboliquement une liaison simple ou une liaison double, procédé caractérisé en ce qu'on fait fermenter l'α-sitostérol

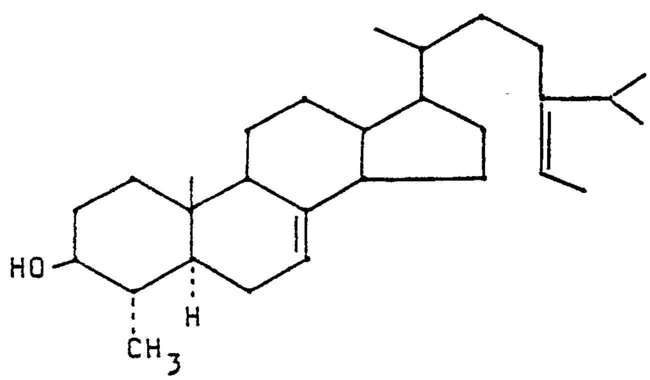

avec une culture d'une souche de micro-organimes capable de dégrader des chaînes latérales de stérols.

2. Procédé pour préparer l'androstène-4 dione-3,17 et l'androstadiène-1,4 dione-3,17 selon la revendication 1, procédé caractérisé en ce qu'on fait fermenter l'α-sitostérol avec une souche de microorganismes du genre Mycobacterium ou Nocardia capable de dégrader des chaînes latérales de stérols.

3. Procédé pour préparer l'androstène-4 dione-3,17 et l'androstadiène-1,4 dione-3,17 selon l'une des revendications 1 et 2, procédé caractérisé en ce qu'on fait fermenter l'α-sitostérol avec Mycobacterium spec. NRRL B-3805, Mycobacterium spec NRRL B-3683, Mycobacterium phlei NRRL B-8154 ou Mycobacterium fortuitum NRRL B-8153.